# EUROPÄISCHE PATENTSCHRIFT

(11) **EP 3 445 218 B1**
(45) Veröffentlichungstag und Bekanntmachung des Hinweises auf die Patenterteilung: **29.01.2020**
(21) Anmeldenummer: 17712783.4
(22) Anmeldetag: 22.03.2017
(51) Int. Cl.: A61B 1/00, G02B 23/24

(54) **ENDOSKOPVORRICHTUNG FÜR EINE AUTOMATISIERTE UNTERSUCHUNG**
ENDOSCOPE DEVICE FOR AN AUTOMATIC EXAMINATION
SYSTÈME D'ENDOSCOPE POUR EXAMEN AUTOMATISÉ

(30) Priorität: 21.04.2016 DE 102016206810
(43) Veröffentlichungstag der Anmeldung: 27.02.2019
(73) Patentinhaber: ZF Friedrichshafen AG, 88046 Friedrichshafen (DE)
(72) Erfinder: WOLF, Daniel, 88048 Friedrichshafen (DE); SCHÄFER, Johannes, 88048 Friedrichshafen (DE); KURUCZ,Marton, 88048 Friedrichshafen (DE)
(86) Internationale Anmeldenummer: PCT/EP2017/056852
(87) Internationale Veröffentlichungsnummer: WO 2017/182222

(56) Entgegenhaltungen:
- JP-A- 2000 089 131
- JP-A- 2005 323 778
- JP-A- 2006 014 959
- JP-A- 2008 224 727

## Beschreibung

Die Erfindung betrifft eine Endoskopvorrichtung zur automatisierten Untersuchung eines Getriebeinnenraums, wobei die Endoskopvorrichtung insbesondere in einem Getriebe einer Windenergieanlage verwendbar ist.

Für Inspektionszwecke von Getrieben wird ein Bild aus dem aus einem Getriebeinnenraum benötigt. Um diese Sicht zu erhalten, ist in der Regel die physische Anwesenheit von Wartungspersonal am Getriebe erforderlich. Zur Vermeidung einer aufwändigen Demontage des Getriebes können über Revisionsöffnungen manuell Endoskope zur Untersuchung des Getriebeinnenraums eingeführt werden. Dies ist insbesondere für Getriebe von Windenergieanlagen zeit- und kostenintensiv.

JP 2008-224727 offenbart eine Endoskopvorrichtung mit einer Steuerungseinheit und einer Vorschubeinheit zur Untersuchung von Industiriemaschinen.

Die DE 10 2009 032 667 A1 offenbart ein Getriebe einer Windenergieanlage, wobei das Getriebe ein Getriebegehäuse aufweist. An der Außenseite des Getriebegehäuses ist wenigstens eine Zugangsöffnung zum Einbringen eines Arbeitsinstruments in das Getriebegehäuse vorgesehen. Im Inneren des Getriebegehäuses ist wenigstens ein Führungsmittel für das Arbeitsinstrument vorgesehen, so dass das Arbeitsinstrument innerhalb des Führungsmittels bewegbar und/oder positionierbar ist. Mittels des im Führungsmittel eingebrachten Arbeitsinstruments werden Teile des Getriebes im Getriebegehäuse geprüft und/oder bearbeitet. Insbesondere werden wenigstens eine physikalische Eigenschaft eines Mediums, insbesondere von Öl, im Getriebegehäuse oder physikalische Eigenschaften von Bauteilen des Getriebes erfasst.

Die Aufgabe der vorliegenden Erfindung besteht darin, eine Endoskopvorrichtung zur automatisierten Untersuchung eines Getriebeinnenraums zu schaffen.

Die Aufgabe wird gelöst durch den Gegenstand von Patentanspruch 1. Bevorzugte Ausführungsformen sind Gegenstand der abhängigen Ansprüche.

Die erfindungsgemäße Endoskopvorrichtung zur automatisierten Untersuchung eines Getriebeinnenraums umfasst eine Steuerungseinheit und eine Auswerteinheit, eine Vorschubeinheit mit einem Schwenkelement, ein Wechselmagazin mit mehreren Führungskanälen sowie ein Endoskop, wobei die Steuerungseinheit mit dem Endoskop verbunden ist und im Wesentlichen zum Steuern des Endoskops sowie zur Weiterleitung von Bildaufnahmen an die Auswerteinheit vorgesehen ist, wobei das Schwenkelement zum Ausrichten eines distalen Endes des Endoskops gegenüber einem jeweiligen Führungskanal vorgesehen ist, und wobei die Vorschubeinheit zum axialen Durchführen des Endoskops durch den jeweiligen Führungskanal vorgesehen ist. Mit anderen Worten ermöglicht die Endoskopvorrichtung eine Sichtung des Getriebeinnenraums aus der Ferne. Die Endoskopvorrichtung wird vorzugsweise über eine dafür vorgesehene Bedieneinrichtung, insbesondere über ein Webinterface gesteuert. Unter einer automatisierten Untersuchung ist eine zumindest teilweise automatisierte Untersuchung zu verstehen. Mit anderen Worten ist zumindest die physische Anwesenheit von Wartungspersonal am Getriebe nicht erforderlich, da der Betrieb der Endoskopvorrichtung automatisiert abläuft. Insbesondere ist dazu die Steuerungseinheit von einem Endgerät, vorzugsweise von einem Rechnersystem steuerbar. Unter einem Rechnersystem ist eine Vorrichtung zu verstehen, die aus physikalischen Komponenten, der sogenannten Hardware, und damit zusammenwirkenden Programmen, der sogenannten Software, besteht. Das Rechnersystem ist vorteilhafterweise als Computer oder als Smartphone ausgebildet und entweder vom Wartungspersonal oder einem Programm steuerbar. Bei einer vollautomatisierten Steuerung wird sowohl die Untersuchung als auch die Auswertung und Zusammenfassung der Untersuchung ohne ein Zutun des Wartungspersonals ausgeführt.

Das Getriebe umfasst ein Getriebegehäuse, das mindestens eine Zugangsöffnung zur Aufnahme der Endoskopvorrichtung aufweist, wobei mittels der Endoskopvorrichtung eine automatisierte Untersuchung des Getriebeinnenraums durchführbar ist. Mithin erfolgt eine Integration der Endoskopvorrichtung im Getriebe. Die Steuerungseinheit ist vorzugsweise kabellos mit der Auswerteeinheit verbunden, wobei die Auswerteeinheit außerhalb des Getriebes angeordnet ist. Insbesondere steuert die Steuerungseinheit nicht nur das Endoskop, insbesondere den Blickwinkel im Getriebeinneren, sondern auch einen Vorschub an der Vorschubeinheit sowie einen Winkel am Schwenkelement, der zur Auswahl des jeweiligen Führungskanals vorgesehen ist. Ferner ist das Wechselmagazin in oder an der Zugangsöffnung angeordnet und ersetzt vorzugsweise einen Revisionsdeckel. Ein jeweiliger Führungskanal im Wechselmagazin ist mit einem jeweiligen Inspektionskanal oder einer jeweiligen Schiene im Getriebegehäuse verbunden, die dazu vorgesehen ist, das Endoskop an die jeweilige zu inspizierende Position, insbesondere einem jeweiligen Zahnrad, einer jeweiligen Welle oder einem jeweiligen Lager zu führen. Mithin kann das Endoskop über das Wechselmagazin automatisiert zwischen den jeweiligen Inspektionskanälen oder Schienen wechseln. Vorzugsweise ist ein Vorschub, eine Rotation und eine Verstellung des distalen Endes des Endoskops motorisch verstellbar. Die Steuerungseinheit ist entweder dezentral oder zentral ausgeführt und steht vorzugsweise mit einer Telemetrieeinheit im Endoskop signaltechnisch in Verbindung.

Vorzugsweise ist mindestens ein Führungskanal mit einer Reinigungsvorrichtung verbunden, um zumindest das distale Ende des Endoskops zu reinigen. Die Reinigungsvorrichtung umfasst ein Reinigungsmittel, das zur Reinigung des Endoskops, insbesondere von Getriebeöl, vorgesehen ist. Ferner kann die Reinigungsvorrichtung darüber hinaus Bürstenelemente, eine Spüleinrichtung, ein Ultraschallbad oder eine Reinigungskassette aufweisen. Vorzugsweise wird das Endoskop beim Herausziehen aus der Reinigungsvorrichtung getrocknet. Dazu ist insbesondere eine Druckluftvorrichtung oder ein Abstreifmittel, insbesondere aus einem Gummi, einem Textil oder einem porösen Material vorgesehen.

Bevorzugt weist das Endoskop an dem distalen Ende eine Kameravorrichtung (Bildsensor) auf. Die Kameravorrichtung ist zur digitalen Bildaufnahme vorgesehen und generiert hochauflösende Foto- oder Videoaufnahmen vom Getriebeinnenraum. Alternativ ist es auch denkbar, dass das Endoskop unterschiedliche optische Elemente, insbesondere Linsen und Spiegel aufweist, um eine Bildaufnahme aus dem Getriebeinnenraum an die Steuerungsvorrichtung zu übertragen. Weiter kann die Kameravorrichtung auch in der Steuereinrichtung enthalten sein, wobei dann ein Lichtwellenleiter zum distalen Ende des Endoskops führt.

Ferner bevorzugt weist das Endoskop an dem distalen Ende eine Beleuchtungsvorrichtung auf. Die Beleuchtungsvorrichtung ist zur Beleuchtung des Getriebeinnenraums vorgesehen und umfasst mindestens eine LED. Insbesondere kann die Beleuchtungsvorrichtung sowohl zum Erzeugen eines Blitzlichtes als auch zur dauerhaften Beleuchtung vorgesehen sein. Das Blitzlicht wird insbesondere zur kurzzeitigen Ausleuchtung des Getriebeinnenraums während einer Fotoaufnahme verwendet. Des Weiteren ist die Kameravorrichtung vorteilhafterweise zur Foto- oder Videoaufnahme in der Dunkelheit vorgesehen, und umfasst mindestens einen Infrarotsensor oder eine Nachtsichtkamera. Foto- oder Videoaufnahmen können in regelmäßigen oder unregelmäßigen Zeitabständen bedarfsgesteuert erfolgen. Vorzugsweise wirkt die Kameravorrichtung mit mindestens einem Sensorelement zur Zustandsüberwachung eines Getriebeelements zusammen, wobei die Kameravorrichtung bei einem kritischen Signal des mindestens einen Sensorelements die Foto- oder Videoaufnahme von dem jeweiligen Getriebeelement startet. Mithin ist auch eine ereignisgesteuerte Foto- oder Videoaufnahme denkbar.

Unter einem kritischen Signal ist insbesondere die Abweichung einer Zustandsgröße von einem zu erwartenden Wert bzw. von einem zu erwartenden Wertebereich zu verstehen. Diese Abweichung kann unterschiedliche Ursachen haben. Ein Beispiel für eine solche Abweichung ist eine Schwingungsänderung durch wachsende Risse in Getriebebauteilen, wie Wellen. Die Ausgabe eines kritischen Signals weist somit auf eine Unstimmigkeit oder einen Fehler des zu überwachenden Getriebeelements hin. Bei der Ausgabe eines kritischen Signals können erforderliche Maßnahmen nach einer visuellen Prüfung mittels der Endoskopvorrichtung eingeleitet werden. Mit anderen Worten ermöglicht die Endoskopvorrichtung eine automatisierte Sichtung des Getriebeinnenraums aus der Ferne.

Des Weiteren bevorzugt sind die Führungskanäle im Wechselmagazin zumindest teilweise kreisförmig oder linear benachbart zueinander angeordnet. Vorzugsweise sind die Führungskanäle in einer Ebene ausgebildet und identisch zu einem Revolvermagazin kreisförmig und somit rotationssymmetrisch angeordnet. Ferner ist es auch denkbar, die Führungskanäle linear benachbart zueinander anzuordnen oder eine Matrix aus Spalten und Zeilen von Führungskanälen auszubilden. Ebenfalls denkbar ist eine dreidimensionale Ausbildung des Wechselmagazins, insbesondere eine kugelförmige oder kugelsegmentförmige Ausbildung.

Gemäß einer bevorzugten Ausführungsform weist der jeweilige Führungskanal einen Sensor zur Positionserfassung des Endoskops auf. Mittels des Sensors ist die genaue Position des distalen Endes des Endoskops bestimmbar. In vorteilhafter Weiterbildung stehen die Steuerungseinheit der Endoskopvorrichtung und eine Steuerungseinheit des Getriebes derart miteinander in Verbindung, dass verschiedene Positionen innerhalb des Getriebes angefahren werden können. Beispielsweise können durch einen Abgleich einer Drehzahl eines Zahnrads die einzelnen Zähne erfasst und zugeordnet werden.

Weiter kann das Getriebe selbst auch Führungskanäle beziehungsweise Führungsschienen aufweisen. Dabei führen die Führungskanäle in dem Getriebe zu den zu inspizierenden Stellen. Die Führungskanäle, welche rotierende Bauteile überbrücken, können ferner fluchtend ausgerichtet sein. In einem Zustand, in welchem durch die Endoskopvorrichtung bewegliche Teile des Getriebes miteinander gekoppelt sind (aufgrund der Durchführung einer Inspektion), muss sichergestellt werden, dass das Getriebe nicht verstellt wird. Andernfalls hätte dies eine Beschädigung der Endoskopvorrichtung zur Folge. Auch hierfür ist es sinnvoll, dass die Steuerungseinheit der Endoskopvorrichtung und die Steuerungseinheit des Getriebes miteinander in Verbindung stehen, beziehungsweise ein Datenaustausch stattfindet.

Insbesondere ist die Auswerteinheit zur Erzeugung eines virtuellen Modells des Getriebeinnenraums vorgesehen. Dabei können die Realbilder zu einem virtuellen Modell zusammengefügt werden, um eine Rundumsicht im Getriebeinnenraum zu ermöglichen. Weiter können Bildaufnahmen überlagert werden, insbesondere aktuelle Realbilder mit virtuellen Modellbildern. Somit kann ein Abgleich mit zwischen Realbildern und Modellbildern stattfinden. Des Weiteren können Zusatzinformationen, wie Bauteilnummern und/oder virtuell nummerierte Zähne von Verzahnungen, eingeblendet werden und Bestandteile des Bedienkonzepts bilden. Insbesondere kann dadurch gezielt eine Zahnflanke eines definierten Zahns mit dem Endoskop angefahren werden und mit archivierten Aufnahmen des jeweiligen Zahns abgeglichen werden. Dazu ist insbesondere ein Positionssensor an der jeweiligen Verzahnung oder Welle vorgesehen, sodass der jeweilige Zahn wieder auffindbar ist. Insbesondere steht die Steuerungseinrichtung der Endoskopvorrichtung mit einer Kontrolleinrichtung des Getriebes beziehungsweise mit den jeweiligen Sensorelementen des Getriebes, insbesondere Sensoren zur Positionsbestimmung und zur Drehwinkelbestimmung in Verbindung. Aufgrund der signaltechnischen Verbindung zwischen der Steuerungseinrichtung der Endoskopvorrichtung und der Kontrolleinrichtung des Getriebes werden Führungen zwischen beweglichen Teilen synchronisiert. Während das Endoskop bewegliche Teile koppelt, insbesondere zwei relativ zueinander bewegliche Teile überdrückt, wird das Getriebe nicht verstellt.

Vorzugsweise ist die Auswerteinheit zur automatisierten Auswertung der Bildaufnahmen vorgesehen. Dazu werden insbesondere die Bildaufnahmen mit Referenzbildern einer Datenbank abgeglichen, wobei die Referenzbilder alle bekannten oder bisher aufgetretenen Schäden oder kritischen Änderungen aufweisen. Diese Auswertung kann insbesondere mit Betriebsmanagementsystemen in Verbindung stehen, von denen eine Inspektion, zum Beispiel aufgrund von Auffälligkeiten einer Zustandsüberwachung, automatisiert veranlasst werden können. Je nach Automatisierungsgrad und Ergebnis der Inspektion können Folgemaßnahmen, wie zum Beispiel der Austausch von Teilen veranlasst oder der Betrieb des Getriebes verändert werden.

Im Folgenden werden Ausführungsbeispiele der Erfindung anhand der Zeichnungen, in welcher gleiche oder ähnliche Elemente mit dem gleichen Bezugszeichen versehen sind, näher erläutert. Hierbei zeigt
- Fig. 1: eine vereinfachte schematische Schnittdarstellung eines teilweise dargestellten Getriebes mit einer erfindungsgemäßen Endoskopvorrichtung zur automatisierten Untersuchung eines Getriebeinnenraums,
- Fig. 2: eine vereinfachte schematische Schnittdarstellung der erfindungsgemäßen Endoskopvorrichtung nach Fig. 1 mit einem Wechselmagazin gemäß einem bevorzugten Ausführungsbeispiel,
- Fig. 3: eine vereinfachte schematische Draufsicht eines erfindungsgemäßen Wechselmagazins nach einem zweiten Ausführungsbeispiel, und
- Fig. 4: eine vereinfachte schematische Draufsicht eines erfindungsgemäßen Wechselmagazins nach einem dritten Ausführungsbeispiel.

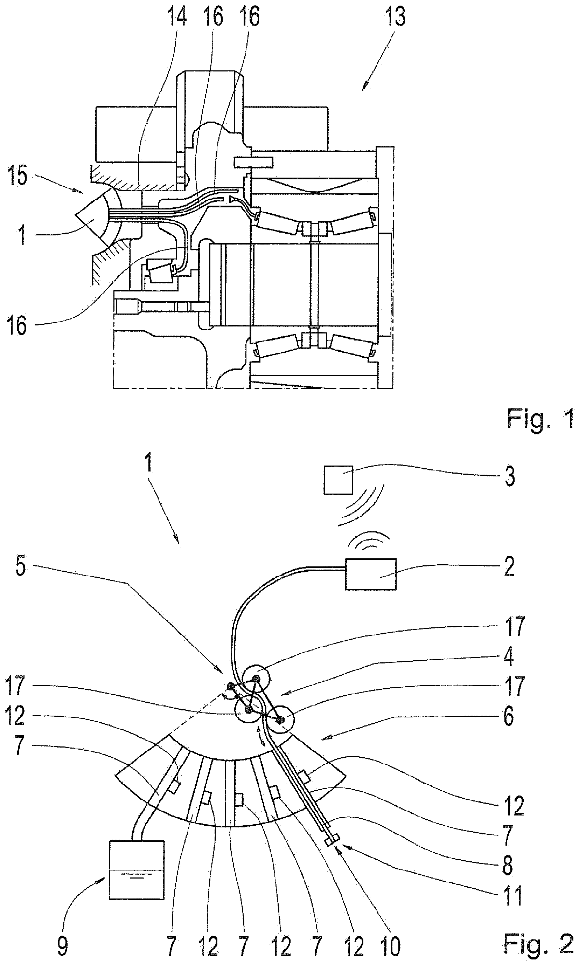

Gemäß Figur 1 weist ein erfindungsgemäßes Getriebe 13 für eine Windenergieanlage ein Getriebegehäuse 14 auf, das eine Zugangsöffnung 15 umfasst. In der Zugangsöffnung 15 ist eine erfindungsgemäße Endoskopvorrichtung 1 angeordnet, wobei mittels der Endoskopvorrichtung 1 eine automatisierte Untersuchung eines Getriebeinnenraums durchführbar ist. Zur Untersuchung verschiedener Getriebebauteile, insbesondere unterschiedlicher Lagerelemente, Wälzkörper, Wellen und Verzahnungen weist das Getriebe 13 mehrere Führungskanäle 7 bzw. Führungsschienen 16 auf, die ein Durchführen eines Endoskops 8 der Endoskopvorrichtung 1an das jeweilige Getriebebauteil ermöglichen.

Nach Figur 2 umfasst die erfindungsgemäße Endoskopvorrichtung 1 gemäß Figur 1 eine Steuerungseinheit 2, eine Vorschubeinheit 4 mit einem Schwenkelement 5, ein Wechselmagazin 6 mit mehreren Führungskanälen 7 sowie das Endoskop 8. Ferner umfasst die Endoskopvorrichtung 1 auch eine Auswerteinheit 3, die außerhalb des Getriebes 13 angeordnet ist und mit der Steuerungseinheit 2 kabellos zusammenwirkt. Insbesondere ist die Auswerteinheit 3 zur Erzeugung eines virtuellen Modells des Getriebeinnenraums vorgesehen, wobei dazu mehrere Bildaufnahmen zu einem virtuellen Modell überlagert werden. Ferner ist die Auswerteinheit 3 auch zur automatisierten Auswertung der Bildaufnahmen vorgesehen.

Die Steuerungseinheit 2 ist dabei mit dem Endoskop 8 verbunden und im Wesentlichen zum Steuern des Endoskops 8 sowie zur Weiterleitung von Bildaufnahmen an die Auswerteinheit 3 vorgesehen. Insbesondere steht das Endoskop 8 in direkter Verbindung zur Steuerungseinheit 2. Das Schwenkelement 5 ist zum Ausrichten eines distalen Endes des Endoskops 8 gegenüber einem jeweiligen Führungskanal 7 und somit zur Auswahl des jeweiligen Führungskanals 7 vorgesehen. Das Wechselmagazin 6 weist mehrere Führungskanäle 7 auf, wobei ein Führungskanal 7 mit einer Reinigungsvorrichtung 9 verbunden ist, um das distale Ende des Endoskops 8 zu reinigen. Insbesondere ist das Wechselmagazin 6 kreissegmentförmig ausgebildet, wobei die Führungskanäle 7 linear benachbart zueinander angeordnet sind. Des Weiteren weist der jeweilige Führungskanal 7 einen Sensor 12 zur Positionserfassung des Endoskops 8 auf. Die Vorschubeinheit 4 ist zum axialen Durchführen des Endoskops 8 durch den jeweiligen Führungskanal 7 bis hin zum jeweiligen Getriebebauteil vorgesehen und weist drei Führungsrollen 17 auf, die an dem Endoskop 8 zur Anlage kommen. Das Schwenkelement 5 ist als drehbare Lagerstelle der Vorschubeinheit 4 ausgebildet und ermöglicht ein Schwenken der Vorschubeinheit 4 um eine Schwenkachse. Je nach Winkelstellung des Schwenkelements 5 wird das distale Ende des Endoskops 8 relativ zum jeweiligen Führungskanal 7 positioniert. Das Endoskop 8 weist an dem distalen Ende eine Kameravorrichtung 10 und eine Beleuchtungsvorrichtung 11 auf.

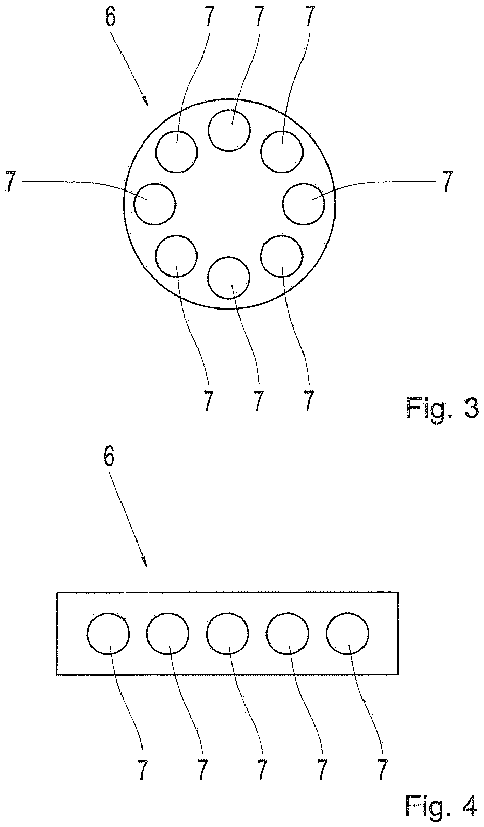

Gemäß Figur 3 ist das Wechselmagazin 6 nach einem alternativen Ausführungsbeispiel dargestellt. Dabei sind die jeweiligen Führungskanäle 7 im Wechselmagazin 6 kreisförmig angeordnet, wobei das Wechselmagazin 6 rotationssymmetrisch ausgebildet ist.

In Figur 4 ist das Wechselmagazin 6 nach einem alternativen Ausführungsbeispiel dargestellt. Dabei sind die jeweiligen Führungskanäle 7 im Wechselmagazin 6 linear benachbart zueinander angeordnet.

### Bezugszeichen

- 1: Sensorvorrichtung
- 2: Steuerungseinheit
- 3: Auswerteinheit
- 4: Vorschubeinheit
- 5: Schwenkelement
- 6: Wechselmagazin
- 7: Führungskanal
- 8: Endoskop
- 9: Reinigungsvorrichtung
- 10: Kameravorrichtung
- 11: Beleuchtungsvorrichtung
- 12: Sensor
- 13: Getriebe
- 14: Getriebegehäuse
- 15: Zugangsöffnung
- 16: Führungsschiene
- 17: Führungsrolle

## Patentansprüche

1. Endoskopvorrichtung (1) zur automatisierten Untersuchung eines Getriebeinnenraums, umfassend eine Steuerungseinheit (2) und eine Auswerteinheit (3), eine Vorschubeinheit (4) mit einem Schwenkelement (5), ein Wechselmagazin (6) mit mehreren Führungskanälen (7) sowie ein Endoskop (8), wobei die Steuerungseinheit (2) mit dem Endoskop (8) verbunden ist und im Wesentlichen zum Steuern des Endoskops (8) sowie zur Weiterleitung von Bildaufnahmen an die Auswerteinheit (3) vorgesehen ist, wobei das Schwenkelement (5) zum Ausrichten eines distalen Endes des Endoskops (8) gegenüber einem jeweiligen Führungskanal (7) vorgesehen ist, und wobei die Vorschubeinheit (4) zum axialen Durchführen des Endoskops (8) durch den jeweiligen Führungskanal (7) vorgesehen ist.

2. Endoskopvorrichtung (1) nach Anspruch 1, wobei mindestens ein Führungskanal (7) mit einer Reinigungsvorrichtung (9) verbunden ist, um zumindest das distale Ende des Endoskops (8) zu reinigen.

3. Endoskopvorrichtung (1) nach einem der vorhergehenden Ansprüche, wobei das Endoskop (8) an dem distalen Ende eine Kameravorrichtung (10) aufweist.

4. Endoskopvorrichtung (1) nach einem der vorhergehenden Ansprüche, wobei das Endoskop (8) an dem distalen Ende eine Beleuchtungsvorrichtung (11) aufweist.

5. Endoskopvorrichtung (1) nach einem der vorhergehenden Ansprüche, wobei die Führungskanäle (7) im Wechselmagazin (6) zumindest teilweise kreisförmig oder linear benachbart zueinander angeordnet sind.

6. Endoskopvorrichtung (1) nach einem der vorhergehenden Ansprüche, wobei der jeweilige Führungskanal (7) einen Sensor (12) zur Positionserfassung des Endoskops (8) aufweist.

7. Endoskopvorrichtung (1) nach einem der vorhergehenden Ansprüche, wobei die Auswerteinheit (3) zur Erzeugung eines virtuellen Modells des Getriebeinnenraums vorgesehen ist.

8. Endoskopvorrichtung (1) nach einem der vorhergehenden Ansprüche, wobei die Auswerteinheit (3) zur automatisierten Auswertung der Bildaufnahmen vorgesehen ist.

9. Verwendung der Endoskopvorrichtung (1) nach einem der Ansprüche 1 bis 8 in einem Getriebe (13) einer Windenergieanlage.

10. Getriebe (13),für eine Windenergieanlage, umfassend ein Getriebegehäuse (14) mit mindestens einer Zugangsöffnung (15) zur Aufnahme einer Endoskopvorrichtung (1) nach einem der Ansprüche 1 bis 8, und eine in der Zugangsöffnung (15) angeordnete Endoskopvorrichtung (1) nach einem der Ansprüche 1 bis 8, wobei mittels der Endoskopvorrichtung (1) eine automatisierte Untersuchung des Getriebeinnenraums durchführbar ist.

## Claims

1. Endoscope device (1) for automated inspection of an interior of a transmission, the endoscope device (1) comprising a control unit (2) and an evaluation unit (3), a feed unit (4) with a swivel element (5), a changer magazine (6) with a plurality of guide channels (7), and an endoscope (8), wherein the control unit (2) is connected to the endoscope (8) and is provided mainly for controlling the endoscope (8) and transmitting images to the evaluation unit (3), wherein the swivel element (5) is provided for aligning a distal end of the endoscope (8) in relation to a respective guide channel (7), and wherein the feed unit (4) is provided for passing the endoscope (8) axially through the respective guide channel (7).

2. Endoscope device (1) according to Claim 1, wherein at least one guide channel (7) is connected to a cleaning device (9) in order to clean at least the distal end of the endoscope (8).

3. Endoscope device (1) according to one of the preceding claims, wherein the endoscope (8) has a camera device (10) at the distal end.

4. Endoscope device (1) according to one of the preceding claims, wherein the endoscope (8) has an illumination device (11) at the distal end.

5. Endoscope device (1) according to one of the preceding claims, wherein the guide channels (7) in the changer magazine (6) are arranged at least partially in a circle or linearly adjacent to each other.

6. Endoscope device (1) according to one of the preceding claims, wherein each guide channel (7) has a sensor (12) for detecting a position of the endoscope (8).

7. Endoscope device (1) according to one of the preceding claims, wherein the evaluation unit (3) is provided for generating a virtual model of the interior of the transmission.

8. Endoscope device (1) according to one of the preceding claims, wherein the evaluation unit (3) is provided for automated evaluation of the images.

9. Use of the endoscope device (1) according to one of Claims 1 to 8 in a transmission (13) of a wind turbine.

10. Transmission (13) for a wind turbine, comprising a transmission housing (14) with at least one access opening (15) for receiving an endoscope device (1) according to one of Claims 1 to 8, and an endoscope device (1) according to one of Claims 1 to 8 arranged in the access opening (15), wherein an automated inspection of the interior of the transmission can be carried out by means of the endoscope device (1).

## Revendications

1. Dispositif endoscopique (1) destiné à l'exploration automatisée d'un espace intérieur de transmission, le dispositif endoscopique comprenant une unité de commande (2) et une unité d'évaluation (3), une unité d'avancement (4) pourvue d'un élément pivotant (5), un magasin interchangeable (6) pourvu d'une pluralité de canaux de guidage (7) et un endoscope (8), l'unité de commande (2) étant reliée à l'endoscope (8) et étant prévue sensiblement pour commander l'endoscope (8) et pour transmettre des enregistrements d'image à l'unité d'évaluation (3), l'élément pivotant (5) étant prévu pour orienter une extrémité distale de l'endoscope (8) par rapport à un canal de guidage respectif (7), et l'unité d'avancement (4) étant prévue pour guider axialement l'endoscope (8) à travers le canal de guidage respectif (7).

2. Dispositif endoscopique (1) selon la revendication 1, au moins un canal de guidage (7) étant relié à un dispositif de nettoyage (9) pour nettoyer au moins l'extrémité distale de l'endoscope (8).

3. Dispositif endoscopique (1) selon l'une des revendications précédentes, l'endoscope (8) comportant à l'extrémité distale un dispositif formant caméra (10) .

4. Dispositif endoscopique (1) selon l'une des revendications précédentes, l'endoscope (8) comportant à l'extrémité distale un dispositif d'éclairage (11).

5. Dispositif endoscopique (1) selon l'une des revendications précédentes, les canaux de guidage (7) ménagés dans le magasin interchangeable (6) étant disposés de manière au moins partiellement circulaire ou de manière linéairement adjacente les uns par rapport aux autres.

6. Dispositif endoscopique (1) selon l'une des revendications précédentes, le canal de guidage respectif (7) comportant un capteur (12) destiné à détecter la position de l'endoscope (8).

7. Dispositif endoscopique (1) selon l'une des revendications précédentes, l'unité d'évaluation (3) étant prévue pour générer un modèle virtuel de l'espace intérieur de la transmission.

8. Dispositif endoscopique (1) selon l'une des revendications précédentes, l'unité d'évaluation (3) étant prévue pour l'évaluation automatisée des enregistrements d'image.

9. Utilisation du dispositif endoscopique (1) selon l'une des revendications 1 à 8 dans une transmission (13) d'une éolienne.

10. Transmission (13) destinée à une éolienne, la transmission comprenant un boîtier de transmission (14) pourvu d'au moins une ouverture d'accès (15) destinée à recevoir un dispositif endoscopique (1) selon l'une des revendications 1 à 8, et un dispositif endoscopique (1) disposé dans l'ouverture d'accès (15) selon l'une des revendications 1 à 8, une exploration automatisée de l'espace intérieur de la transmission pouvant être effectuée au moyen du dispositif endoscopique (1).
